(19) Europäisches Patentamt
European Patent Office
Office européen des brevets

(11) **EP 3 173 191 B1**

(12) **EUROPEAN PATENT SPECIFICATION**

(45) Date of publication and mention
of the grant of the patent:
**17.03.2021 Bulletin 2021/11**

(51) Int Cl.:
***B25J 9/00*** *(2006.01)*     ***A61F 2/70*** *(2006.01)*
***A61H 1/02*** *(2006.01)*     ***A61H 3/00*** *(2006.01)*

(21) Application number: **16180661.7**

(22) Date of filing: **21.07.2016**

(54) **METHOD FOR ESTIMATING POSTURE OF ROBOTIC WALKING AID**

VERFAHREN ZUR SCHÄTZUNG DER HALTUNG EINER ROBOTISCHEN GEHHILFE

PROCÉDÉ D'ESTIMATION DE POSTURE DE ROBOTIQUE D'AIDE À LA MARCHE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2015 TW 104139717**

(43) Date of publication of application:
**31.05.2017 Bulletin 2017/22**

(73) Proprietor: **Industrial Technology Research Institute**
**Chu-Tung, Hsin-Chu (TW)**

(72) Inventors:
• **SUN, KUAN-CHUN**
**64345 Yunlin County (TW)**
• **TSAI, YI-JENG**
**330 Taoyuan City (TW)**
• **WU, CHENG-HUA**
**30046 Hsinchu City (TW)**
• **HU, JWU-SHENG**
**30274 Hsinchu County (TW)**

(74) Representative: **Lang, Christian**
**LangPatent Anwaltskanzlei IP Law Firm**
**Ingolstädter Straße 5**
**80807 München (DE)**

(56) References cited:
**WO-A1-2014/164804**     **WO-A2-2010/027968**
**CN-A- 103 622 792**     **US-A1- 2006 130 594**
**US-B1- 7 190 141**

• **Bruno Siciliano, and Oussama Khatib: "Springer Handbook of Robotics", 27 June 2016 (2016-06-27), Springer ISBN: 978-3-319-32550-7 pages 1203-1234, DOI: 978-3-319-32550-7, * Section 48.3 ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The present disclosure relates to a method for estimating posture of a robotic walking aid, and more particularly, to a method for estimating posture of a robotic walking aid for further use in remote service.

**BACKGROUND**

Considering the global trend of aging population and low fertility rate, many countries had already suffered a serious shortage of manpower, and thus had to postpone their statutory retirement age. For overcoming such manpower shortage, many developed countries have invested many resources into the development and integration of robotic technology and information-and-communication technology (ICT) for producing industrial robots to be used in many automation applications, such as a robotic walking aid which is especially being commonly configured in a form of exoskeleton robot system. It is noted that a good robotic walking aid not only can have reducing the working load of manual labor, but also can be used for providing quality assurance of long-term care and walking assistance to the elderly. By enabling a robotic walking aid to detect the body movement of a user, the robotic walking aid is able to provide power to assist the body movement, so that the overall power supporting the body movement of the user is increased, and in some extreme case, the robotic walking aid can even help a paralyzed user to stand up. Nowadays, the most commercially successful and commonly used robotic walking aid is the lower-limb exoskeleton robot, such as the RewalkTM by Argo Medical Technologies in Israel, the EksoTM by Ekso Biobics in U.S.A., the HAL by Cyberdyne in Japan, the ITRI-EXO by ITEI in Taiwan, and the Stride Management Assist Device by Honda in Japan. Generally, with the help of a walking stick or other waling aids, a robotic walking aid can help a user to accomplish many ritual activities in his/her daily life, such as getting up, sitting down, walking uphill and downhill, and walking upstairs and downstairs. Since such robotic waling aids are designed for people with difficulties in mobility and for elderly people, safety concern in usage is the most important issue and that is also the essential part of study in the development of robotic walking aid that is lack of technological support.

In one prior art, there is already a safety mechanism available for determining when the user is in a safe position from which to take a step. Operationally, posture information of a user is obtained using measurements of angular orientation with respect to gravity provided by an inertial measurement unit that is mounted on a shank of the user, and pressure center of the user is calculated and obtained using a pressure sensor that is arranged at the sole of a foot, and then the posture information and the calculated pressure center arc used in a calculation for determining whether the user is in a safe position from which to take a step. In another prior art, there is another safety mechanism for determining when the user is in a safe position from which to take a step, in which measurements of angular orientation with respect to gravity are provided by the use of some angular sensors that are affixed to the trunk of a user while the angular orientation measurements are used for determining whether the user is in a safe position from which to take a step.

Nevertheless, in prior arts or other disclosed research documentations, there is no applications employing aforesaid information in remote service, which may include topography feedback, danger prompting, falling alert and distress call, exercise amount estimation, walking distance estimation, behavior monitoring, activity record, rehabilitation feedback, and so on.

Therefore, the robotic walking aids in prior arts still have many imperfections. US 2006/0130594 A1 and US 7,190,141 B1 disclose walking aids whose balance is monitored on the basis of a two-dimensional calculation. WO 2010/027968 A2 discloses a method for controlling a walking aid according to which terrain texture irregularities are determined and the walking aid is dynamically controlled on the basis of such information.

**SUMMARY**

According to the invention a method is provided having the features of claim 1. Further embodiments are subject-matter of the dependent claims.

Further scope of applicability of the present application will become more apparent from the detailed description given hereinafter. However, it should be understood that the detailed description and specific examples, while indicating exemplary embodiments of the disclosure, are given by way of illustration only. The scope of the invention is defined by the attached claims and will become apparent to those skilled in the art from this detailed description.

**BRIEF DESCRIPTION OF THE DRAWINGS**

The present disclosure will become more fully understood from the detailed description given herein below and the accompanying drawings which are given by way of illustration only, and thus are not limitative of the present disclosure and wherein:

FIG. 1 is a flow chart depicting steps performed in a method for estimating posture of a robotic walking aid according to an embodiment of the present disclosure.
FIG. 2 is a schematic diagram showing a framework of a robotic walking aid of the present disclosure.
FIG. 3 is a schematic diagram showing a mathematical model of a robotic walking aid of the present disclosure.
FIG. 4 is a flow chart depicting steps for determining whether the posture and 3D coordinates of the center of gravity are abnormal in the present disclosure.

## DETAILED DESCRIPTION

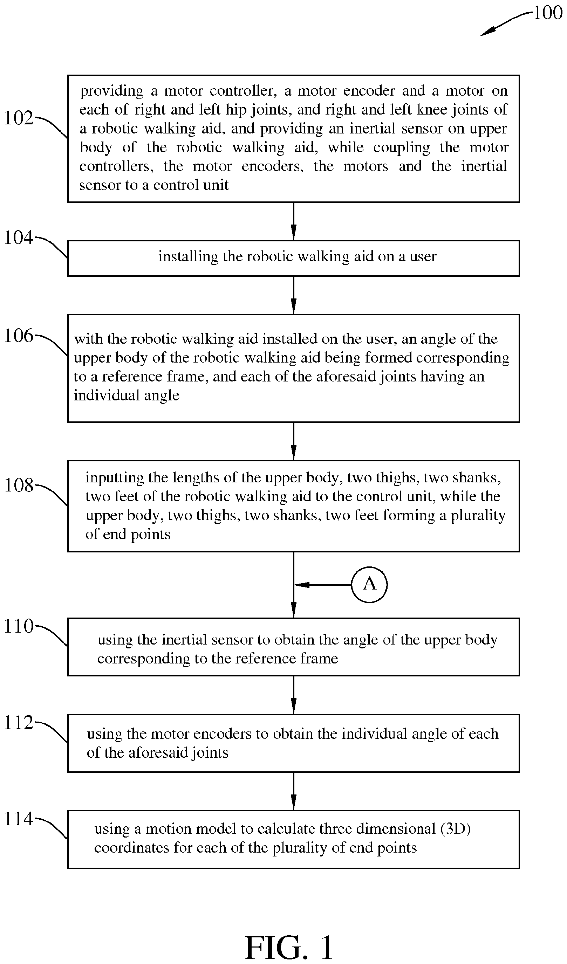

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing. Please refer to FIG. 1, which is a flow chart depicting steps performed in a method for estimating posture of a robotic walking aid according to the present invention. The method 100 of FIG. 1 comprises the following steps:

step 102: providing a motor controller, a motor encoder and a motor on each of right and left hip joints, and right and left knee joints of a robotic walking aid, and providing an inertial sensor on upper body of the robotic walking aid, while coupling the motor controllers, the motor encoders, the motors and the inertial sensor to a control unit;
step 104: installing the robotic walking aid on a user;
step 106: with the robotic walking aid installed on the user, an angle of the upper body of the robotic walking aid being formed corresponding to a reference frame, and each of the aforesaid joints having an individual angle;
step 108: inputting the lengths of the upper body, two thighs, two shanks, two feet of the robotic walking aid to the control unit, while the upper body, two thighs, two shanks, two feet forming a plurality of end points;
step 110: using the inertial sensor to obtain the angle of the upper body corresponding to the reference frame;
step 112: using the motor encoders to obtain the individual angle of each of the aforesaid joints; and
step 114: using a motion model to calculate three dimensional (3D) coordinates for each of the plurality of end points.

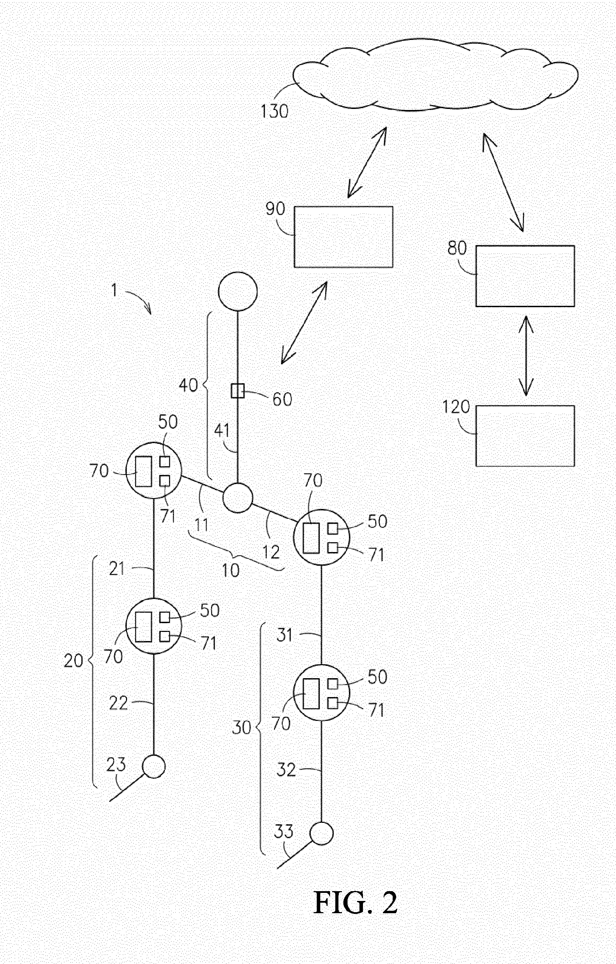

Please refer to FIG. 2, which is a schematic diagram showing a framework of a robotic walking aid of the present disclosure. In FIG. 2, the robotic walking aid 1 includes: a pelvis 10, a right leg 20, left leg 30 and an upper body 40. The pelvis 10 is composed of a first link 11 and a second link 12 that are serially connected to each other. The right leg 20 is composed of a third link 21, a fourth link 22, and a fifth link 23, that are arranged serially connected to one another while allowing the right leg 20 to couple to one end of the pelvis 10 in a form that the node between the right leg 20 and the pelvis 10 is defined to be the right hip joint, the node between the third link 21 and the fourth link 22 is defined to be the right knee joint, whereas the third link 21 is defined to be the right thigh, the fourth link 22 is defined to be the right shank and the fifth link 23 is defined to be the right foot. Similarly, the left leg 30 is composed of a sixth link 31, a seventh link 32, and an eighth link 33, that are arranged serially connected to one another while allowing the left leg 30 to couple to another end of the pelvis 10 not connecting to the right leg 20 in a form that the node between the left leg 30 and the pelvis 10 is defined to be the left hip joint, the node between the sixth link 31 and the seventh link 32 is defined to be the left knee joint, whereas the sixth link 31 is defined to be the left thigh, the seventh link 32 is defined to be the left shank and the eighth link 33 is defined to be the left foot. Moreover, there is further a ninth link 41 that is being disposed for enabling one end thereof to connect to the node between the first link 11 and the second link 12 so as to be used as the upper body 40 of the robotic walking aid 1. Except for motors, there can be some kinds of hydraulic or artificial muscle actuators being disposed at those joints.

According to the invention, at each of the right and left hip joints, and the right and left knee joints of a robotic walking aid 1, there are a motor controller 50, a motor encoder 71 and a motor 70 to be mounted respectively thereat; and there is an inertial sensor 60 to be mounted on the upper body 40 of the robotic walking aid 1, whereas the motor controllers 50, the motor encoders 71, the motors 70 and the inertial sensor 60 are coupled to a control unit 80. Thereby, by the use of a cloud computing means 130, the robotic walking aid 1 is able to connect to a mobile communication device 90 via the control unit 80. It is noted that the mobile communication device 90 can be a smart phone, a tablet computer or a smart watch, whichever is built with GPS function. Therefore, the mobile communication device 90 is able to for provide the GPS coordinates of the user with the robotic walking aid 1 installed on so as to be used in an activity motoring, and moreover, while working cooperatively with the inertial sensor 60, the mobile communication device 90 can provide indoor positioning information for monitoring any user wearing the mobile communication device 90. In addition, the control unit 80 is further connected to a database 120 for allowing information to be transmitted between the control unit 80, the mobile communication device 90 and the database 120 via the cloud computing means 130 for future used in remote service, and the remote service can include a topography feedback process, a danger prompting process, a falling alert and distress call process, an exercise amount estimation process, a walking distance estimation process, a behavior monitoring process, an activity record process, and a rehabilitation feedback process. The robotic walking aid 1 is designed to be worn by a user for helping the user to walk. Moreover, there can be a power source for providing power to the robotic walking aid 1.

Although the control unit 80 is connected to the robotic walking aid 1 in a wireless manner in this embodiment, it can be connected to the robotic walking aid 1 in a wired manner, or in another embodiment, the control unit 80 can be installed directly on the robotic walking aid 1.

The inertial sensor 60 can be the composition of an accelerometer, a gyroscope, a magnetometer, and an angle gauge, whichever can perform a posture estimation algorithm for estimating upper body 40 posture of a user, estimating walking steps of a user and for indoor positioning, and so on. In an embodiment, the inertial sensor 60 is a 9-degree-of-freedom inertial measurement unit (9D IMU), which is generally an assembly including a three-axis accelerometer, a gyroscope and a magnetometer, and is used for estimating an inertial motion of an object, or for calculating a transformation matrix for the coordinate of the inertial sensor 60 corresponding to a reference coordinate system. The accelerometer is a device that will measure acceleration forces, whereas these forces may be static, like the constant force of gravity, or they could be dynamic, caused by moving or vibrating the accelerometer. The gyroscope is a device capable of measuring the angular rate of an object, and while working cooperatively with an accelerometer, the gyroscope can measure moment of inertial that is not detectable by the accelerometer, so that the dimension of detection as well as the system frequency are enhanced. The magnetometer is a device capable of direction of the magnetic field at a point in space, and can be used as an electronic compass that can work cooperatively with an accelerometer and a gyroscope for estimating a yaw angle of an object.

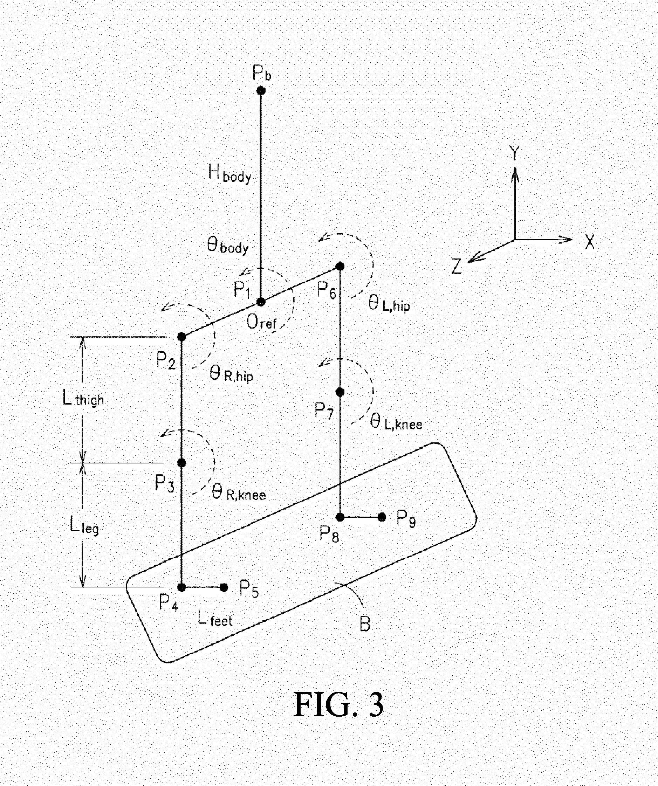

Please refer to FIG. 3, which is a schematic diagram showing a mathematical model of a robotic walking aid 1 of the present disclosure. The mathematical model of FIG. 3 is established based upon the robotic walking aid 1 of FIG. 2. In FIG. 3, one end of the ninth link 41 that is connected to the node between the first link 11 and the second link 12 is defined to be a first end point $P_1$; another end of the ninth link 41 that is opposite to the first end point $P_1$ is defined to be an upper body end point $P_b$; an end of the third link 21 that is connected to the first link 11 is defined to be a second end point $P_2$; another end of the third link 21 that is connected to the fourth link 22 is defined to be a third end point $P_3$; an end of the fourth link 22 that is connected to the fifth link 23 is defined to be a fourth end point $P_4$; an end of the fifth link 23 that is disposed corresponding to the fourth end point $P_4$ is defined to be a fifth end point $P_5$; an end of the sixth link 31 that is connected to the seventh link 32 is defined to be a seventh end point $P_7$; an end of the seventh link 32 that is connected to the eighth link 33 is defined to be an eighth end point $P_8$; an end of the eighth link 33 that is disposed corresponding to the eighth end point $P_8$ is defined to be a ninth end point $P_9$.

In the following description, the step 114 of FIG. 1 for using the motion model to calculate a spatial coordinate for each end point is described with reference to FIG. 2 and FIG. 3. The x-axis, y-axis and z-axis of a reference frame is provided and shown in FIG. 3, whereas a direction that the user is walking toward is defined to be the positive direction of an x-axis in the reference frame; the node between the upper body 40 and the pelvis 10 of the robotic walking aid 1 is defined to be the origin $O_{ref}$ of the reference frame while respectively allowing the first end point $P_1$ to the ninth end point $P_9$ to be the origins of a sub-coordinate frame *1* to a sub-coordinate frame *9* and the upper body end point $P_b$ to be the origin of a sub-coordinate frame *0*; and consequently the 3D coordinates of each of the first end point $P_1$ to the ninth end point $P_9$ and the upper body end point $P_b$ corresponding to the reference frame can be obtained by a homogeneous transformation matrixes defined by the plurality of the aforesaid end points. It is noted that the transformation relationship between two neighboring sub-coordinate frames can be represented by $R_{ij}$, in which $R_{ij}$ is substantially a transformation matrix for the transformation from the sub-coordinate frame *j* to the sub-coordinate frame *i*, and can be defined as following:

$$R_{r1} = \begin{bmatrix} \cos\theta_{body} & -\sin\theta_{body} & 0 & 0 \\ \sin\theta_{body} & \cos\theta_{body} & 0 & 0 \\ 0 & 0 & 1 & 0 \\ 0 & 0 & 0 & 1 \end{bmatrix} \quad R_{12} = \begin{bmatrix} \cos\theta_{R,hip} & -\sin\theta_{R,hip} & 0 & 0 \\ \sin\theta_{R,hip} & \cos\theta_{R,hip} & 0 & 0 \\ 0 & 0 & 1 & W_{waist} \\ 0 & 0 & 0 & 1 \end{bmatrix}$$

$$R_{23} = \begin{bmatrix} \cos\theta_{R,knee} & -\sin\theta_{R,knee} & 0 & 0 \\ \sin\theta_{R,knee} & \cos\theta_{R,knee} & 0 & -L_{thihg} \\ 0 & 0 & 1 & 0 \\ 0 & 0 & 0 & 1 \end{bmatrix} \qquad R_{34} = \begin{bmatrix} \cos 0 & -\sin 0 & 0 & 0 \\ \sin 0 & \cos 0 & 0 & -L_{leg} \\ 0 & 0 & 1 & 0 \\ 0 & 0 & 0 & 1 \end{bmatrix}$$

$$R_{45} = \begin{bmatrix} \cos 0 & -\sin 0 & 0 & L_{feet} \\ \sin 0 & \cos 0 & 0 & 0 \\ 0 & 0 & 1 & 0 \\ 0 & 0 & 0 & 1 \end{bmatrix} \qquad R_{16} = \begin{bmatrix} \cos\theta_{L,hip} & -\sin\theta_{L,hip} & 0 & 0 \\ \sin\theta_{L,hip} & \cos\theta_{L,hip} & 0 & 0 \\ 0 & 0 & 1 & -W_{waist} \\ 0 & 0 & 0 & 1 \end{bmatrix}$$

$$R_{67} = \begin{bmatrix} \cos\theta_{L,knee} & -\sin\theta_{L,knee} & 0 & 0 \\ \sin\theta_{L,knee} & \cos\theta_{L,knee} & 0 & -L_{thihg} \\ 0 & 0 & 1 & 0 \\ 0 & 0 & 0 & 1 \end{bmatrix} \qquad R_{78} = \begin{bmatrix} \cos 0 & -\sin 0 & 0 & 0 \\ \sin 0 & \cos 0 & 0 & -L_{leg} \\ 0 & 0 & 1 & 0 \\ 0 & 0 & 0 & 1 \end{bmatrix}$$

$$R_{89} = \begin{bmatrix} \cos 0 & -\sin 0 & 0 & L_{feet} \\ \sin 0 & \cos 0 & 0 & 0 \\ 0 & 0 & 1 & 0 \\ 0 & 0 & 0 & 1 \end{bmatrix}$$

Consequently, $R_{r1}$ is substantially a transformation matrix for the transformation from sub-coordinate frame *1* to the reference frame; $R_{12}$ is substantially a transformation matrix for the transformation from the sub-coordinate frame *2* to the sub-coordinate frame *1*, $R_{23}$ is substantially a transformation matrix for the transformation from the sub-coordinate frame *3* to the sub-coordinate frame *2*, $R_{34}$ is substantially a transformation matrix for the transformation from the sub-coordinate frame *4* to the sub-coordinate frame *3*, $R_{45}$ is substantially a transformation matrix for the transformation from the sub-coordinate frame *5* to the sub-coordinate frame *4*, $R_{16}$ is substantially a transformation matrix for the transformation from the sub-coordinate frame *6* to the sub-coordinate frame *1*, $R_{67}$ is substantially a transformation matrix for the transformation from the sub-coordinate frame *7* to the sub-coordinate frame *6*, $R_{78}$ is substantially a transformation matrix for the transformation from the sub-coordinate frame *8* to the sub-coordinate frame *7*, and $R_{89}$ is substantially a transformation matrix for the transformation from the sub-coordinate frame *9* to the sub-coordinate frame *8*.

For instance, for obtaining the transformation relationship of the third end point $P_3$ corresponding to the reference frame, it can be obtained using the following formula:

$$p_3 = R_{r1} \cdot R_{12} \cdot R_{23} \cdot \begin{bmatrix} 0 & 0 & 0 & 1 \end{bmatrix}^T ;$$

and similarly, the other end points can be obtained in the following formulas:

$$p_b = R_{r1} \cdot \begin{bmatrix} 0 & H_{body} & 0 & 1 \end{bmatrix}^T$$

$$p_1 = R_{r1} \cdot \begin{bmatrix} 0 & 0 & 0 & 1 \end{bmatrix}^T$$

$$p_2 = R_{r1} \cdot R_{12} \cdot \begin{bmatrix} 0 & 0 & 0 & 1 \end{bmatrix}^T$$

$$p_4 = R_{r1} \cdot R_{12} \cdot R_{23} \cdot R_{34} \cdot \begin{bmatrix} 0 & 0 & 0 & 1 \end{bmatrix}^T$$

$$p_5 = R_{r1} \cdot R_{12} \cdot R_{23} \cdot R_{34} \cdot R_{45} \cdot \begin{bmatrix} 0 & 0 & 0 & 1 \end{bmatrix}^T$$

$$p_6 = R_{r1} \cdot R_{16} \cdot \begin{bmatrix} 0 & 0 & 0 & 1 \end{bmatrix}^T$$

$$p_7 = R_{r1} \cdot R_{16} \cdot R_{67} \cdot \begin{bmatrix} 0 & 0 & 0 & 1 \end{bmatrix}^T$$

$$p_8 = R_{r1} \cdot R_{16} \cdot R_{67} \cdot R_{78} \cdot \begin{bmatrix} 0 & 0 & 0 & 1 \end{bmatrix}^T$$

$$p_9 = R_{r1} \cdot R_{16} \cdot R_{67} \cdot R_{78} \cdot R_{89} \cdot \begin{bmatrix} 0 & 0 & 0 & 1 \end{bmatrix}^T$$

Therefore, as shown in FIG. 3, the posture of the robotic walking aid 1 can be estimated after inputting the roll and pitch relating to the joints and upper body of the robotic walking aid 1, which includes $\theta_{body}$, $\theta_{R,hip}$, $\theta_{R, knee}$, $\theta_{L, hip}$, $\theta_{L, knee}$. Moreover, a base of support $B$ can be constructed by projecting a plane formed by the connection of the fourth end point $P_4$, the fifth end point $P_5$, the eighth end point $P_8$, and the ninth end point $P_9$. Thereafter, the mass center of the user wearing the robotic walking aid 1 can be calculated. First, the center coordinates of those links are obtained using the following formulas:

$$c_{body} = \frac{p_b + p_1}{2} \quad ,$$

$$c_{R,thigh} = \frac{p_2 + p_3}{2} \quad ,$$

$$c_{R,leg} = \frac{p_3 + p_4}{2} \quad ,$$

$$c_{R,feet} = \frac{p_4 + p_5}{2} \quad ,$$

$$c_{L,thigh} = \frac{p_6 + p_7}{2} \quad ,$$

$$c_{L,leg} = \frac{p_7 + p_8}{2} \quad ,$$

$$c_{L,feet} = \frac{p_8 + p_9}{2} \quad .$$

Then, after obtaining the ratio of each linkage rate corresponding to the overall weight of the user by the use of the Dempster's coefficient, the spatial coordinate corresponding to the center of gravity of the robotic walking aid 1 can be obtained as following:

$$CoM = \frac{\sum c_i \cdot m_i}{\sum m_i}$$

.

Consequently, the center of gravity is projected to the base of support **B** for obtaining the required spatial relationship.

In addition, after the angular orientation of the user with respect to gravity is detected and provided by the inertial sensor 60, the motion model can be modified accordingly and then to be used in mapping marks via the GPS positioning function of the mobile communication device 90 for next user.

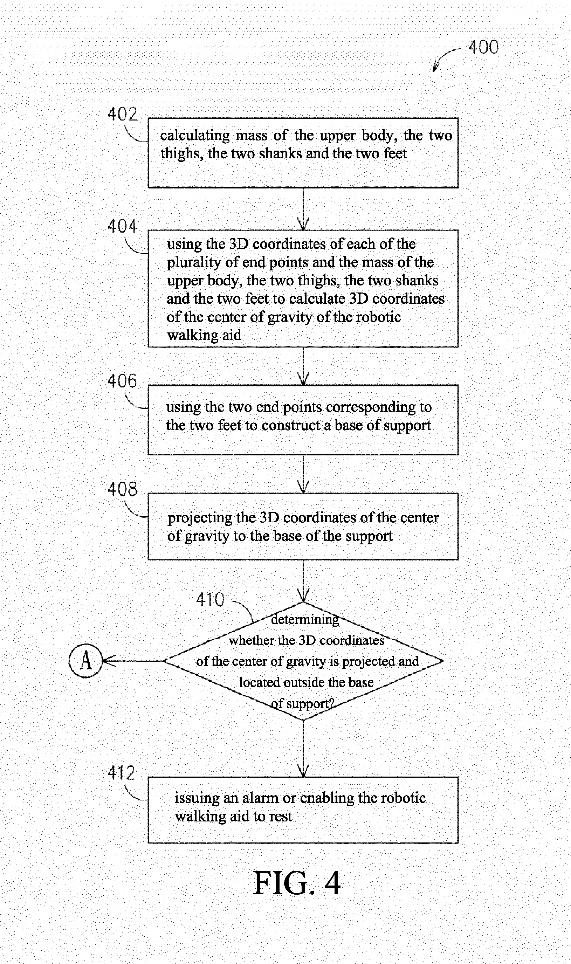

Accordingly, after the using of the motion model to calculate a spatial coordinate for each end point is performed, as disclosed in FIG. 1, a process for determining whether the posture and the 3D coordinates of the center of the gravity of the user are abnormal can be concluded and obtained. Please refer to FIG. 4, which is a flow chart depicting steps for determining whether the posture and 3D coordinates of the center of gravity are abnormal in the present disclosure. In FIG. 4, the process 400 comprises the following steps:

step 402:    calculating mass of the upper body 40, the two thighs, the two shanks and the two feet;

step 404:    using the 3D coordinates of each of the plurality of end points and the mass of the upper body 40, the two thighs, the two shanks and the two feet to calculate 3D coordinates of the center of gravity of the robotic walking aid 1;

step 406:    using the two end points corresponding to the two feet to construct a base of support;

step 408:    projecting the 3D coordinates of the center of gravity to the base of the support; and

step 410:    determining whether the 3D coordinates of the center of gravity is projected and located outside the base of support; and if so, issuing an alarm for enabling the robotic walking aid 1 to rest at step 412; otherwise, returning to the step of using the inertial sensor 60 to obtain the angle of the upper body 40 corresponding to the reference frame, as indicated by the point A in FIG. 1 and FIG. 4.

The steps performed in FIG. 4 can be divided into two processes, in which the steps 402 ~ 404 is a process for calculating 3D coordinates of the center of gravity of the user; and the steps 406 ~ 412 is a process for determining whether the 3D coordinates of the center of gravity of the user are abnormal.

In FIG. 2, the control unit 80 is connected to a mobile communication device 90 with GPS function and a database 120. Operationally, the mobile communication device 90 with GPS function is enabled to detect and transmit the GPS coordinates of the robotic walking aid 1 along with the angles relating to the upper body 40 and the joints of the robotic walking aid 1 to the database 120 to be used in remote service. Moreover, the remote service includes a topography feedback process, a danger prompting process, a falling alert and distress call process, an exercise amount estimation process, a walking distance estimation process, a behavior monitoring process, an activity record process, and a rehabilitation feedback process.

In the topography feedback process, the GPS coordinates of the user is matched to a map, e.g. Google map, for identifying terrains of specific topographic marks, and when a user approaches any of those specific topographic marks, a remote prompting is issued for suggesting the user to alter his/her walking mode for adapting to the terrain of the approached topographic mark.

In the danger prompting process, the GPS coordinates of the user is matched to a map, e.g. Google map, for identifying dangerous locations, and when a user approaches any of those dangerous locations, a remote prompting is issued for alerting the user to cope with the coming danger.

In the falling alert and distress call process, the posture of the user is obtained using the angles relating to the upper body 40 and the aforesaid joints of the robotic walking aid 1, and when the posture is determined to be abnormal, a call is made to find out the condition of the user, and if there is no response from the user, an active distress call is issued to an emergency medical unit that is located nearest to the user according to the GPS coordinates of the user.

In the exercise amount estimation process, an exercise amount is calculated and obtained using the flowing formula:

$$(m_r + m_h) \times g \times d = W_r + W_h;$$

wherein $m_r$ is the mass of the robotic walking aid 1;

$g$ is the gravitational acceleration;

$m_h$ is the mass of the user;

$d$ is the walking distance;

$W_r$ is the mechanic energy generated by the robotic walking aid 1;

$W_h$ is the physical cost of the user, i.e. the exercise amount of the user.

It is noted that the masses of the robotic walking aid 1 and the user can be obtained by any common weight measurement device, and the walking distance can be estimated and obtained according to the information detected by the inertial sensor 60 relating to the amount of walking steps of the robotic walking aid 1. In addition, the mechanical energy consumed by the robotic walking aid 1 can be estimated according to the battery residual capacity. However, the energy conversion efficiency for converting electrical energy into mechanical energy must be identified first, and then mechanical energy consumed by the robotic walking aid 1 can be estimated according to the battery residual capacity accordingly. After obtaining the mechanical energy consumed by the robotic walking aid 1, the physiological cost of the user can be calculated by the use of the aforesaid formula. Therefore, in this embodiment, the energy conversion efficiency for converting electrical energy into mechanical energy is identified as following:

$$W_r = W_{mechanical} = \eta\, W_{electrical};$$

wherein $W_{mechanical}$ is the mechanical energy generated by the robotic walking aid 1;
$W_{electrical}$ is the electrical energy consumed by the robotic walking aid 1; and
$\eta$ is the conversion efficiency.

Moreover, the exercise amount can be estimated by the use of a vision-based motion analysis system, such as the VICON motion analysis system that is operated cooperatively with a force plate. During the estimation of the exercise amount, the overall energy consumed in the movement, including the kinetic energy and potential energy, is calculated, and a physiological cost measurement is performed by the use of a oxygen consumption measurement device, such as Cosmed K2, and thereby, an energy conversion efficiency database for the robotic walking aid 1 under various walking conditions can be established so as to be used in the exercise amount calculation. Thus, the exercise amount can be obtained using the following formula:

$$W_h = (m_r + m_h) \times g \times d - \eta\, W_{electrical}$$

In the walking distance estimation process, a posture of the user is obtained remotely using the angles relating to the upper body 40 and the aforesaid joints of the robotic walking aid 1 and a step length of the user is estimated so as to be used for estimating and recording the walking distance.
In the behavior monitoring process, postures of the user are obtained remotely using the angles relating to the upper body 40 and the aforesaid joints of the robotic walking aid 1, and the postures of the user are classified into different behaviors according to a classification rule to be recorded.
In the activity record process, the GPS coordinates of the user are matched to a map, e.g. Google map, for identifying and recording places where the user perform his/her daily activities.
In the rehabilitation feedback process, the postures, step length, step frequency, and exercise amount are recorded and provided remotely to a rehabilitation therapist for constructing a rehabilitation treatment accordingly.
To sum up, the present disclosure provides a method for estimating posture of a robotic walking aid, using which a safety control and instant posture adjustment mechanism for the robotic walking aid are enabled via the cooperation between inertial sensor and motor encoders; an indoor and outdoor GPS positioning can be achieved via the communication between the inertial sensors and a mobile communication device, while allowing the result of the GPS positioning to be provided to an remote service center for monitoring and behavior analysis. Consequently, the remote service center can decide whether to provide a remote service operation accordingly, and the remote service operation includes a topography feedback process, a danger prompting process, a falling alert and distress call process, an exercise amount estimation process, a walking distance estimation process, a behavior monitoring process, an activity record process, and a rehabilitation feedback process.

With respect to the above description then, it is to be realized that the optimum dimensional relationships for the parts of the disclosure, to include variations in size, materials, shape, form, function and manner of operation, assembly and use, are deemed readily apparent and obvious to one skilled in the art.

**Claims**

1. A method for estimating posture of a robotic walking aid (1), comprising the steps of:

providing a motor controller (50), a motor encoder (71) and a motor (70) on each of right and left hip joints, and right and left knee joints of the robotic walking aid (1), and providing an inertial sensor (60) on upper body (40) of the robotic walking aid (1), while coupling the motor controllers (50), the motor encoders (71), the motors (70) and the inertial sensor (60) to a control unit (80);

installing the robotic walking aid (1) on a user;

with the robotic walking aid (1) installed on the user, an angle of the upper body (40) of the robotic walking aid (1) being formed corresponding to a reference frame, and each of the aforesaid joints having an individual angle;

inputting the lengths of the upper body (40), two thighs, two shanks, two feet of the robotic walking aid (1) to the control unit, while the upper body (40), two thighs, two shanks, two feet forming a plurality of end points;

using the inertial sensor (60) to obtain the angle of the upper body corresponding to the reference frame;

using the motor encoders (71) to obtain the individual angle of each of the aforesaid joints; and

using a motion model to calculate three dimensional (3D) coordinates for each of the plurality of end points, wherein after the step of using of the motion model to calculate 3D coordinates for each of the plurality of end points is performed, a process for calculating 3D coordinates of the center of gravity of the user wearing the walking aid (1) is performed, and the process comprises the steps of:

calculating mass of the upper body (40), the two thighs, the two shanks and the two feet; and

using the 3D coordinates of each of the plurality of end points and the mass of the upper body, the two thighs, the two shanks and the two feet to calculate 3D coordinates of the center of gravity of the robotic walking aid (1), wherein after the center of gravity of the user wearing the robotic walking aid (1) is obtained, a process for determining whether the 3D coordinates of the center of gravity of the user wearing the robotic walking aid is abnormal is performed, and the process comprises the steps of:

using the two end points corresponding to the two feet to construct a base of support (B);

projecting the 3D coordinates of the center of gravity to the base of the support (B); and

determining whether the 3D coordinates of the center of gravity is projected and located outside the base of support (B); and if so, issuing an alarm or enabling the robotic walking aid (1) to rest; otherwise, returning to the step of using the inertial sensor (60) to obtain the angle of the upper body (40) corresponding to the reference frame,

wherein the robotic walking aid (1) is defined by a plurality of links, and the plurality of links include:

a first link (11) and a second link (12), serially connected to each other to compose a pelvis (10);

a third link (21), a fourth link (22), and a fifth link (23), serially connected to one another to compose a right leg (20) while allowing the right leg (20) to couple to one end of the pelvis (10) in a form that a node between the right leg (20) and the pelvis (10) is defined to be the right hip joint, a node between the third link (21) and the fourth link (22) is defined to be the right knee joint, and the fifth link (23) is defined to be the right foot;

a sixth link (31), a seventh link (32), and an eighth link (33), serially connected to one another to compose a left leg (30) while allowing the left leg (30) to couple to one end of the pelvis (10) that is not connected to the right leg (20) in a form that a node between the left leg (30) and the pelvis (10) is defined to be the left hip joint, a node between the sixth link (31) and the seventh link (32) is defined to be the left knee joint, and the eighth link (33) is defined to be the left foot; and

a ninth link (41), used as the upper body (40) and one end thereof being connected to a node between the first link (11) and the second link (12).

2. The method of claim 1, wherein the plurality of end points include:

a first end point, disposed at an end of the ninth link (41) that is connected to the first link (11) and the second link (12);

an upper body (40) end point, disposed at an end of the ninth link (41) that is opposite to the first end point;

a second end point, disposed at an end of the third link (21) that is connected to the first link (11);

a third end point, disposed at an end of the third link (21) that is connected to the fourth link (22);

a fourth end point, disposed at an end of the fourth link (22) that is connected to the fifth link (23);

a fifth end point, disposed at an end of the fifth link (23) that is opposite to the fourth end point;

a sixth end point, disposed at an end of the sixth link (31) that is connected to the second link (12);

a seventh end point, disposed at an end of the sixth link (31) that is connected to the seventh link (32);

an eighth end point, disposed at an end of the seventh link (32) that is connected to the eighth link (33); and

a ninth end point, disposed at an end of the eighth link (33) that is opposite to the eighth end point.

3. The method of claim 2, wherein in the step of using the motion model to calculate 3D coordinates for each of the plurality of end points, a direction that the user is walking toward is defined to be the positive direction of an x-axis in the reference frame; the node between the upper body (40) and the pelvis (10) of the robotic walking aid (1) is defined to be the origin of the reference frame while respectively defining the first end point to the ninth end point to be the origins of a sub-coordinate frame *1* to a sub-coordinate frame 9 and the upper body (40) end point to be the origin of a sub-coordinate frame *0*; and consequently the 3D coordinates of each of the first end point to the ninth end point and the upper body (40) end point corresponding to the reference frame can be obtained by a homogeneous transformation matrix defined by the plurality of the aforesaid end points.

**Patentansprüche**

1. Verfahren zur Abschätzung der Haltung einer robotischen Gehhilfe (1), welches die folgenden Schritte umfasst:

Bereitstellen einer Motorsteuerung (50), eines Motorencoders (71) und eines Motors (70) an jeweils dem rechten und linken Hüftgelenk und rechten und linken Kniegelenk der robotischen Gehhilfe (1) und Bereitstellen eines Trägheitssensors (60) an dem Oberkörper (40) der robotischen Gehhilfe (1), wobei die Motorsteuerungen (50), die Motorencoder (71), die Motoren (70) und der Trägheitssensor (60) mit einer Steuereinheit (80) gekoppelt werden;

Anbringen der robotischen Gehhilfe (1) an einem Benutzer, wobei, wenn die robotische Gehhilfe (1) an dem Benutzer angebracht ist, ein Winkel des Oberkörpers (40) der robotischen Gehhilfe (1) entsprechend einem Bezugssystem gebildet wird, und jedes der zuvor genannten Gelenke einen individuellen Winkel aufweist;

Eingeben der Längen des Oberkörpers (40), der zwei Oberschenkel, der zwei Unterschenkel, der zwei Füße der robotischen Gehhilfe (1) in die Steuereinheit, während der Oberkörper (40), die zwei Oberschenkel, die zwei Unterschenkel, die zwei Füße mehrere Endpunkte bilden;

Verwenden des Trägheitssensors (60), um den Winkel des Oberkörpers zu erhalten, welcher dem Bezugssystem entspricht;

Verwenden der Motorencoder (71), um den individuellen Winkel jedes der zuvor genannten Gelenke zu erhalten; und

Verwenden eines Bewegungsmodells, um dreidimensionale (3D) Koordinaten für jeden der mehreren Endpunkte zu berechnen,

wobei, nachdem der Schritt des Verwendens des Bewegungsmodells zum Berechnen von 3D-Koordinaten für jeden der mehreren Endpunkte durchgeführt wurde, ein Verfahren zum Berechnen von 3D-Koordinaten des Schwerpunktes des Benutzers, welcher die Gehhilfe (1) trägt, durchgeführt wird, und das Verfahren die folgenden Schritte umfasst:

Berechnen der Masse des Oberkörpers (40), der zwei Oberschenkel, der zwei Unterschenkel und der zwei Füße; und

Verwenden der 3D-Koordinaten jedes der mehreren Endpunkte und der Masse des Oberkörpers, der zwei Oberschenkel, der zwei Unterschenkel und der zwei Füße, um 3D-Koordinaten des Schwerpunktes der robotischen Gehhilfe (1) zu berechnen,

wobei, nachdem der Schwerpunkt des Benutzers, welcher die robotische Gehhilfe (1) trägt, erhalten wurde, ein Verfahren zum Bestimmen, ob die 3D-Koordinaten des Schwerpunktes des Benutzers, welcher die robotische Gehhilfe trägt, anormal sind, durchgeführt wird, und das Verfahren die folgenden Schritte umfasst:

> Verwenden der zwei Endpunkte, welche den zwei Füßen entsprechen, um eine Unterstützungsfläche (B) zu erstellen;
> Projizieren der 3D-Koordinaten des Schwerpunktes auf die Unterstützungsfläche (B); und
> Bestimmen, ob die 3D-Koordinaten des Schwerpunktes projiziert werden und
> sich außerhalb der Unterstützungsfläche (B) befinden; und wenn ja, Abgeben eines Alarms oder Ermöglichen, dass die Robotergehhilfe (1) eine Pause macht;
> andernfalls Zurückkehren zu dem Schritt des Verwendens des Trägheitssensors (60), um den Winkel des Oberkörpers (40) zu erhalten, welcher dem Bezugssystem entspricht,
> wobei die robotische Gehhilfe (1) durch mehrere Glieder definiert ist, und die mehreren Glieder Folgendes einschließen:

> > ein erstes Glied (11) und ein zweites Glied (12), die in Reihe miteinander verbunden sind, um ein Becken (10) auszubilden;
> > ein drittes Glied (21), ein viertes Glied (22) und ein fünftes Glied (23), die in Reihe miteinander verbunden sind, um ein rechtes Bein (20) auszubilden, während es dem rechten Bein (20) ermöglicht wird, mit einem Ende des Beckens (10) auf eine Weise zu koppeln, dass ein Knotenpunkt zwischen dem rechten Bein (20) und dem Becken (10) als das rechte Hüftgelenk definiert wird, wobei ein Knotenpunkt zwischen dem dritten Glied (21) und dem vierten Glied (22) als das rechte Kniegelenk definiert wird, und das fünfte Glied (23) als der rechte Fuß definiert wird;
> > ein sechstes Glied (31), ein siebtes Glied (32) und ein achtes Glied (33), die in Reihe miteinander verbunden sind, um ein linkes Bein (30) auszubilden, während es dem linken Bein (30) ermöglicht wird, mit einem Ende des Beckens (10), das nicht mit dem rechten Bein (20) verbunden ist, auf eine Weise zu koppeln, dass ein Knotenpunkt zwischen dem linken Bein (30) und dem Becken (10) als das linke Hüftgelenk definiert wird, wobei ein Knotenpunkt zwischen dem sechsten Glied (31) und dem siebten Glied (32) als das linke Kniegelenk definiert wird und das achte Glied (33) als der linke Fuß definiert wird; und
> > ein neuntes Glied (41), das als der Oberkörper (40) verwendet wird, und wobei ein Ende davon mit einem Knotenpunkt zwischen dem ersten Glied (11) und dem zweiten Glied (12) verbunden ist.

2. Verfahren nach Anspruch 1, wobei die mehreren Endpunkte Folgendes einschließen:

> einen ersten Endpunkt, der an einem Ende des neunten Gliedes (41) angeordnet ist, das mit dem ersten Glied (11) und dem zweiten Glied (12) verbunden ist;
> einen Endpunkt des Oberkörpers (40), der an einem Ende des neunten Gliedes (41) angeordnet ist, das dem ersten Endpunkt gegenüberliegt;
> einen zweiten Endpunkt, der an einem Ende des dritten Gliedes (21) angeordnet ist, das mit dem ersten Glied (11) verbunden ist;
> einen dritten Endpunkt, der an einem Ende des dritten Gliedes (21) angeordnet ist, das mit dem vierten Glied (22) verbunden ist;
> einen vierten Endpunkt, der an einem Ende des vierten Gliedes (22) angeordnet ist, das mit dem fünften Glied (23) verbunden ist;
> einen fünften Endpunkt, der an einem Ende des fünften Gliedes (23) angeordnet ist, das dem vierten Endpunkt gegenüberliegt;
> einen sechsten Endpunkt, der an einem Ende des sechsten Gliedes (31) angeordnet ist, das mit dem zweiten Glied (12) verbunden ist;
> einen siebten Endpunkt, der an einem Ende des sechsten Gliedes (31) angeordnet ist, das mit dem siebten Glied (32) verbunden ist;
> einen achten Endpunkt, der an einem Ende des siebten Gliedes (32) angeordnet ist, das mit dem achten Glied (33) verbunden ist; und
> einen neunten Endpunkt, der an einem Ende des achten Gliedes (33) angeordnet ist, das dem achten Endpunkt gegenüberliegt.

3. Verfahren nach Anspruch 2, wobei in dem Schritt des Verwendens des Bewegungsmodells zum Berechnen von 3D-Koordinaten für jeden der mehreren Endpunkte eine Richtung, in welche der Benutzer geht, als die positive Richtung einer X-Achse in dem Bezugssystem definiert wird; der Knotenpunkt zwischen dem Oberkörper (40) und dem Becken (10) der robotischen Gehhilfe (1) als der Ursprung des Referenzsystems definiert wird, während jeweils der erste Endpunkt bis zu dem neunten Endpunkt als die Ursprünge eines Sub-Koordinatensystems *1* bis zu einem Sub-Koordinatensystem *9* und der Endpunkt des Oberkörpers (40) als der Ursprung eines Sub-Koordinatensystems *0* definiert wird; und folglich die 3D-Koordinaten jedes des ersten Endpunktes bis zu dem neunten Endpunkt und der Endpunkt des Oberkörpers (40), welche dem Bezugsystem entsprechen, durch eine homogene Transformationsmatrix erhalten werden können, die durch die mehreren der zuvor genannten Endpunkte definiert wird.

**Revendications**

1. Procédé pour estimer une posture d'une aide robotique à la marche (1), comprenant les étapes consistant:

à fournir un contrôleur de moteur (50), un codeur de moteur (71) et un moteur (70) sur chacune des articulation gauche et droite de la hanche et articulations gauche et droite du genou de l'aide robotique à la marche (1) et à fournir un capteur inertiel (60) sur un corps supérieur (40) de l'aide robotique à la marche (1) tout en couplant les contrôleurs de moteur (50), les codeurs de moteur (71), les moteurs (70) et le capteur inertiel (60) à une unité de commande (80);
à installer l'aide robotique à la marche (1) sur un utilisateur;
avec l'aide robotique à la marche (1) installée sur l'utilisateur, un angle du corps supérieur (40) de l'aide robotique à la marche (1) étant formé de manière correspondante à un cadre de référence et chacune des articulations susmentionnées ayant un angle individuel;
à entrer les longueurs du corps supérieur (40), les deux cuisses, les deux jambes, les deux pieds de l'aide robotique à la marche (1) dans l'unité de commande pendant que le corps supérieur (40), les deux cuisses, les deux jambes, les pieds forment une pluralité de points d'extrémité;
à utiliser le capteur inertiel (60) pour obtenir l'angle du corps supérieur correspondant au cadre de référence;
à utiliser les codeurs de moteur (71) pour obtenir l'angle individuel de chacune des articulaires susmentionnées; et
à utiliser un modèle de mouvement pour calculer des coordonnées tridimensionnelles (3D) pour chaque point d'extrémité de la pluralité de points d'extrémité,
dans lequel, après l'étape d'utilisation du modèle de mouvement pour calculer des coordonnées 3D pour chaque point d'extrémité de la pluralité de points d'extrémité est réalisée, un processus pour calculer des coordonnées 3D du centre de gravité de l'utilisateur portant l'aide à la marche (1) est effectué et le processus comprend les étapes consistant:

à calculer la masse du corps supérieur (40), les deux cuisses, les deux jambes et les deux pieds; et
à utiliser les coordonnées 3D de chaque point d'extrémité de la pluralité de points d'extrémité et la masse du corps supérieur, des deux cuisses, des deux jambes et des deux pieds pour calculer des coordonnées 3D du centre de gravité de l'aide robotique à la marche (1),
dans lequel, après que le centre de gravité de l'utilisateur portant l'aide robotique à la marche (1) est obtenu, un processus pour déterminer si les coordonnées 3D du centre de gravité de l'utilisateur portant l'aide robotique à la marche sont anormales, est effectué et le processus comprend les étapes consistant:

à utiliser les deux points d'extrémité correspondant aux deux pieds pour construire une base de support (B);
à projeter les coordonnées 3D du centre de gravité sur la base du support (B); et
à déterminer si les coordonnées 3D du centre de gravité sont projetées et localisées à l'extérieur de la base du support (B) ; et si tel est le cas, à émettre une alarme ou à permettre à l'aide robotique à la marche (1) de rester ; sinon, à revenir à l'étape d'utilisation du capteur inertiel (60) pour obtenir l'angle du corps supérieur (40) correspondant au cadre de référence,
dans lequel l'aide robotique à la marche (1) est définie par une pluralité de pièces de liaison et la pluralité de pièces de liaison incluent:

une première pièce de liaison (11) et une deuxième pièce de liaison (12), raccordées en série l'une à l'autre pour composer un pelvis (10);
une troisième pièce de liaison (21), une quatrième pièce de liaison (22) et une cinquième pièce de liaison (23), raccordées en série les unes aux autres pour composer une jambe droite (20) tout en permettant à la jambe droite (20) d'être couplée à une extrémité du pelvis (10) dans une forme montrant qu'un nœud entre la jambe droite (20) et le pelvis (10) est défini pour être l'articulation de la hanche droite, qu'un nœud entre la troisième pièce de liaison (21) et la quatrième pièce de liaison (22) est défini pour être l'articulation du genou droit et que la cinquième pièce de liaison (23) est définie pour être le pied droit;

une sixième pièce de liaison (31), une septième pièce de liaison (32) et une huitième pièce de liaison (33), raccordées en série les unes aux autres pour composer une jambe gauche (30) tout en permettant à la jambe gauche (30) d'être couplée à une extrémité du pelvis (10) qui n'est pas raccordé à la jambe droite (20) dans une forme montrant qu'un nœud entre la jambe gauche (30) et le pelvis (10) est défini pour être l'articulation de la hanche gauche, qu'un nœud entre la sixième pièce de liaison (31) et la septième pièce de liaison (32) est défini pour être l'articulation du genou gauche et que la huitième pièce de liaison (33) est définie pour être le pied gauche; et

une neuvième pièce de liaison (41), utilisée comme corps supérieur (40) et une extrémité de celle-ci étant raccordée à un nœud entre la première pièce de liaison (11) et la deuxième pièce de liaison (12).

2. Procédé selon la revendication 1, dans lequel la pluralité de points d'extrémité incluent:

un premier point d'extrémité, disposé au niveau d'une extrémité de la neuvième pièce de liaison (41) qui est raccordée à la première pièce de liaison (11) et à la deuxième pièce de liaison (12);
un point d'extrémité de corps supérieur (40), disposé au niveau d'une extrémité de la neuvième pièce de liaison (41) qui est opposée au premier point d'extrémité;
un deuxième point d'extrémité, disposé au niveau d'une extrémité de la troisième pièce de liaison (21) qui est raccordée à la première pièce de liaison (11);
un troisième point d'extrémité, disposé au niveau d'une extrémité de la troisième pièce de liaison (21) qui est raccordée à la quatrième pièce de liaison (22);
un quatrième point d'extrémité, disposé au niveau d'une extrémité de la quatrième pièce de liaison (22) qui est raccordée à la cinquième pièce de liaison (23);
un cinquième point d'extrémité, disposé au niveau d'une extrémité de la cinquième pièce de liaison (23) qui est opposée au quatrième point d'extrémité;
un sixième point d'extrémité, disposé au niveau d'une extrémité de la sixième pièce de liaison (31) qui est raccordée à la deuxième pièce de liaison (12);
un septième point d'extrémité, disposé au niveau d'une extrémité de la sixième pièce de liaison (31) qui est raccordée à la septième pièce de liaison (32);
un huitième point d'extrémité, disposé au niveau d'une extrémité de la septième pièce de liaison (32) qui est raccordée à la huitième pièce de liaison (33); et
un neuvième point d'extrémité, disposé au niveau d'une extrémité de la huitième pièce de liaison (33) qui est opposée au huitième point d'extrémité.

3. Procédé selon la revendication 2, dans lequel, à l'étape d'utilisation du modèle de mouvement pour calculer des coordonnées 3D pour chaque point d'extrémité de la pluralité de points d'extrémité, une direction vers laquelle l'utilisation marche, est définie pour être la direction positive d'un axe X dans le cadre de référence ; le nœud entre le corps supérieur (40) et le pelvis (10) de l'aide robotique à la marche (1) est défini pour être l'origine du cadre de référence tout en définissant respectivement le premier point d'extrémité au neuvième point d'extrémité pour être les origines d'un cadre de sous-coordonnées *1* à un cadre de sous-coordonnées *9* et le point d'extrémité de corps supérieur (40) pour être l'origine d'un cadre de sous-coordonnée *0* ; et, par conséquent, les coordonnées 3D de chacun du premier point d'extrémité au neuvième point d'extrémité et du point d'extrémité de corps supérieur (40) correspondant au cadre de référence peuvent être obtenues par une matrice de transformation homogène définie par la pluralité des points d'extrémité susmentionnés.

100

102 — providing a motor controller, a motor encoder and a motor on each of right and left hip joints, and right and left knee joints of a robotic walking aid, and providing an inertial sensor on upper body of the robotic walking aid, while coupling the motor controllers, the motor encoders, the motors and the inertial sensor to a control unit

104 — installing the robotic walking aid on a user

106 — with the robotic walking aid installed on the user, an angle of the upper body of the robotic walking aid being formed corresponding to a reference frame, and each of the aforesaid joints having an individual angle

108 — inputting the lengths of the upper body, two thighs, two shanks, two feet of the robotic walking aid to the control unit, while the upper body, two thighs, two shanks, two feet forming a plurality of end points

(A)

110 — using the inertial sensor to obtain the angle of the upper body corresponding to the reference frame

112 — using the motor encoders to obtain the individual angle of each of the aforesaid joints

114 — using a motion model to calculate three dimensional (3D) coordinates for each of the plurality of end points

# FIG. 1

FIG. 2

FIG. 3

400

402 — calculating mass of the upper body, the two thighs, the two shanks and the two feet

404 — using the 3D coordinates of each of the plurality of end points and the mass of the upper body, the two thighs, the two shanks and the two feet to calculate 3D coordinates of the center of gravity of the robotic walking aid

406 — using the two end points corresponding to the two feet to construct a base of support

408 — projecting the 3D coordinates of the center of gravity to the base of the support

410 — determining whether the 3D coordinates of the center of gravity is projected and located outside the base of support?

A

412 — issuing an alarm or enabling the robotic walking aid to rest

## FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060130594 A1
- US 7190141 B1
- WO 2010027968 A2